# EUROPÄISCHE PATENTSCHRIFT

(11) **EP 4 403 563 B1**
(45) Veröffentlichungstag und Bekanntmachung des Hinweises auf die Patenterteilung: **21.05.2025**
(21) Anmeldenummer: 23152178.2
(22) Anmeldetag: 18.01.2023
(51) Int. Cl.: C07F 9/6574, C07C 45/50, C07C 47/02

(54) **DIPHOSPHIT MIT EINEM ME-FLÜGELBAUSTEIN UND EINEM TBU-FLÜGELBAUSTEIN**
DIPHOSPHITE WITH A ME-WING COMPONENT AND A TBU-WING COMPONENT
DIPHOSPHITE DOTÉE D'UN COMPOSANT À LAME ME ET D'UN COMPOSANT À LAME TBU

(43) Veröffentlichungstag der Anmeldung: 24.07.2024
(73) Patentinhaber: Evonik Oxeno GmbH & Co. KG, 45772 Marl (DE)
(72) Erfinder: SALE, Anna Chiara, 45657 Recklinghausen (DE); FRANKE, Robert, 45772 Marl (DE); FRIDAG, Dirk, 45721 Haltern am See (DE); KUCMIERCZYK, Peter, 44628 Herne (DE); MARKOVIC, Ana, 45721 Haltern am See (DE)
(74) Vertreter: Evonik Patent Association

(56) Entgegenhaltungen:
- CN-B- 103 467 517
- CN-U- 215 196 872

## Beschreibung

Die Erfindung betrifft ein Diphosphit mit einem Me-Flügelbaustein und einem *^{t}*Bu-Flügelbaustein und dessen Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

In CN 215 196 872 U werden Diphosphite und deren Verwendung in der Hydroformylierung beschrieben.

In CN 103 467 517 B wird ein Verfahren zur Herstellung eines Diphosphits beschrieben.

Die technische Aufgabe der Erfindung ist die Bereitstellung einer Verbindung, mit welcher bei der Hydroformylierung von Olefinen eine gesteigerte Ausbeute erzielt werden kann.

Die Aufgabe wird gelöst durch eine Verbindung gemäß Anspruch 1.

Verbindung, welche die Struktur (1) aufweist:

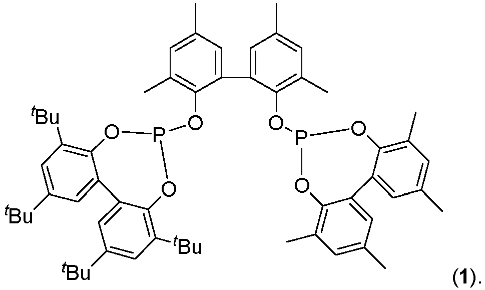

Neben der Verbindung selbst wird auch ein Verfahren beansprucht, in dem die zuvor beschriebene Verbindung zum Einsatz kommt.

Verfahren umfassend die Verfahrensschritte:
a) Vorlegen eines Olefins;
b) Zugabe der zuvor beschriebenen Verbindung;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Substanz, welche Rh umfasst, ausgewählt aus: Rh(acac)(CO)₂, [(acac)Rh(COD)] (acac = Acetylacetonat-Anion; COD = 1,5-Cyclooctadien), Rh₄CO₁₂.

In einer Variante des Verfahrens ist die Substanz, welche Rh umfasst, Rh(acac)(CO)₂.

Im Folgenden soll die Erfindung anhand eines Ausführungsbeispieles näher erläutert werden.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das Substrat (1-Octen) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 100 ppm Rh(acac)(CO)₂ und die entsprechende Menge an Ligand (L:Rh = 50:1) eingewogen und mit 8,0 ml Toluol aufgefüllt. Die jeweils eingebrachte Masse an Toluol wurde für die GC-Analyse bestimmt. Anschließend wurden 1,80 g 1-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 17 bar erhöht und die Reaktion bei konstantem Druck für 1 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

Der Versuch wurde mit den Verbindungen **(1)** und **(2)** durchgeführt.

Der Versuch mit der Verbindung **(2)** dient hierbei als Vergleichsversuch.

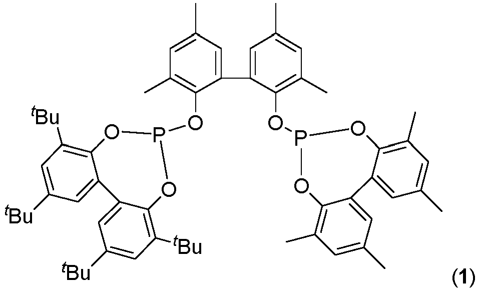

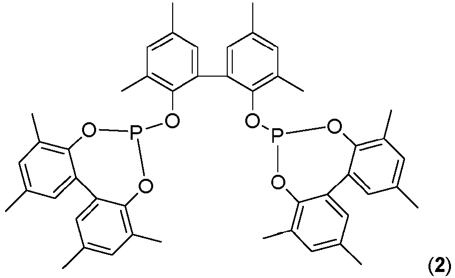

### Ergebnisse der Katalyseversuche

[Rh]: 100 ppm, p: 17 bar, T: 120 °C; t: 1 h

**Tabelle: Hydroformylierung von 1-Octen**

| | Ausbeute [%] |
|---|---|
| **(1)*** | 18 |
| **(2)** | 11 |

| | |
|---|---|
| *erfindungsgemäße Verbindung | |

Die durchgeführten Versuche belegen, dass die gestellte Aufgabe durch eine erfindungsgemäße Verbindung gelöst wird.

## Patentansprüche

1. Verbindung, welche die Struktur **(1)** aufweist:

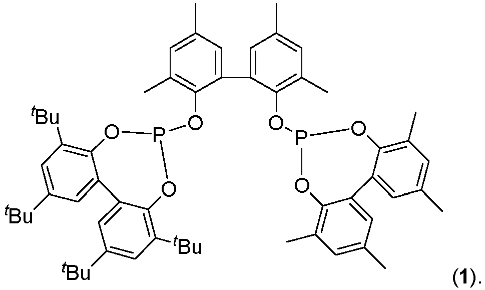

2. Verfahren umfassend die Verfahrensschritte:
a) Vorlegen eines Olefins;
b) Zugabe einer Verbindung nach Anspruch 1;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

3. Verfahren nach Anspruch 2,
wobei die Substanz, welche Rh umfasst, ausgewählt ist aus: Rh(acac)(CO)₂, [(acac)Rh(COD)] (acac = Acetylacetonat-Anion; COD = 1,5-Cyclooctadien), Rh₄CO₁₂.

4. Verfahren nach einem der Ansprüche 2 oder 3,
wobei die Substanz, welche Rh umfasst, Rh(acac)(CO)₂ ist.

## Claims

1. Compound having the structure (1):

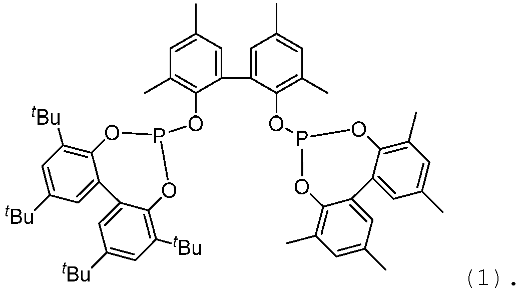

2. Process comprising the process steps of:
a) initially charging an olefin;
b) adding a compound according to Claim 1;
c) adding a substance comprising Rh;
d) feeding in H₂ and CO;
e) heating the reaction mixture from a) to d), to convert the olefin to an aldehyde.

3. Process according to Claim 2,
wherein the substance comprising Rh is selected from: Rh(acac) (CO)₂, [(acac)Rh(COD)] (acac = acetylacetonate anion; COD = 1,5-cyclooctadiene), Rh₄CO₁₂.

4. Process according to either of Claims 2 and 3, wherein the substance comprising Rh is Rh(acac) (CO)₂.

## Revendications

1. Composé qui présente la structure (1) :

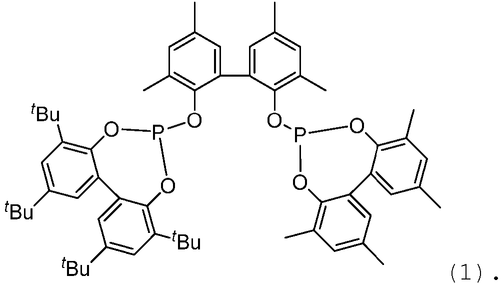

2. Procédé comprenant les étapes de procédé :
a) disposition préalable d'une oléfine ;
b) ajout d'un composé selon la revendication 1 ;
c) ajout d'une substance qui comprend du Rh ;
d) introduction de H₂ et de CO ;
e) chauffage du mélange réactionnel provenant de a) à d), l'oléfine étant convertie en un aldéhyde.

3. Procédé selon la revendication 2,
la substance qui comprend du Rh étant choisie parmi : Rh(acac)(CO)₂, [(acac) Rh(COD)] (acac = anion d'acétylacétonate ; COD = 1,5-cyclooctadiène), Rh₄CO₁₂.

4. Procédé selon l'une des revendications 2 ou 3,
la substance qui comprend du Rh étant Rh(acac) (CO)₂.
